# EUROPEAN PATENT APPLICATION

(11) **EP 4 579 244 A1**
(43) Date of publication of application: **02.07.2025**
(21) Application number: 23857796.9
(22) Date of filing: 25.08.2023
(51) Int. Cl.: G01N 35/00, G01N 35/04

(54) **CONTAINER POSITION INDICATING DEVICE AND ASSEMBLY-TYPE DIAGNOSTIC SYSTEM USING SAME**

(30) Priority: 25.08.2022 KR 20220107027
(71) Applicant: Seegene, Inc., Seoul 05548 (KR)
(72) Inventor: CHANG, Jin A, Seoul 05632 (KR); RO, Moon Yong, Seoul 05544 (KR)
(74) Representative: Viering, Jentschura & Partner mbB Patent- und Rechtsanwälte
(86) International application number: PCT/KR2023/012683
(87) International publication number: WO 2024/043764

(57) **Abstract**

In accordance with an aspect of the present disclosure, there is provided a modular molecular diagnostic system comprising: a preparation device for performing a preparation work for detection of a target nucleic acid molecule; a detection device for performing a detection work for the target nucleic acid molecule; and a display unit that displays a device mark representing the preparation device and a device mark representing the detection device, wherein the display unit displays information about the status of a device represented by each device mark or information about a task that each device performs in a predetermined sequence on device marks.

## Description

### TECHNICAL FIELD

The present disclosure relates to a vessel position display device and a modular diagnostic system using same.

### BACKGROUND ART

In molecular diagnostic tests for disease diagnosis, processes such as nucleic acid extraction, reaction solution setup, amplification reaction, and reaction analysis are performed. Various devices are used at each step.

For example, a nucleic acid extraction device for extracting nucleic acids from a specimen, a preparation device for preparing an analysis sample (e.g., a liquid dispensing device, a reaction solution setup device, etc.), and a detection device for performing an amplification reaction and an analysis of the amplification reaction (e.g., a real-time PCR device, etc.) may be used. In addition, nucleic acid extraction and reaction solution setup may be performed in different devices, or in the same device.

These various devices may be stand-alone. As a result, a user needs to load vessels for reagents, specimens and/or analysis samples for each test step into each device or to move them from one device to another.

External contamination may occur when vessels for specimens, reagents, and/or analysis samples are loaded into each device or transferred to another device by the user. In addition, human testing errors by the user may occur. User intervention may also cause time delays in the aforementioned processes.

To solve these problems, various systems have been developed. One of the systems is a modular molecular diagnostic system in which the aforementioned stand-alone devices are assembled.

### DETAILED DESCRIPTION OF INVENTION

### TECHNICAL PROBLEMS

The objectives to be achieved in one embodiment include a technology for displaying the position of a sample vessel that receives an analysis sample, which is utilized in a modular molecular diagnostic system including the aforementioned reaction preparation device and detection device.

### TECHNICAL SOLUTION

In accordance with an aspect of the present disclosure, there is provided a device for displaying a position of a vessel that accommodates an analysis sample, the device comprising: a display unit; a processor; and a memory, wherein the memory stores at least one instruction, executed by the processor to cause the display unit to display the position of the vessel by positioning a vessel symbol representing the vessel on one of a plurality of device marks, and wherein the plurality of device marks include a device mark representing a preparation device that performs a preparation work for detecting a target nucleic acid molecule of the analysis sample and a device mark representing a detection device that performs a detection work for detecting the target nucleic acid molecule.

Wherein each of the plurality of device marks may include a plurality of task locations indicating a plurality of tasks that each of devices represented by the plurality of device marks performs in accordance with a predetermined sequence, and the processor may execute the at least one instruction to cause the display unit to display the position of the vessel by positioning the vessel symbol at one of the plurality of task locations.

Wherein the plurality of task locations included in the device mark representing the preparation device may include a task location indicating at least one task among a task of generating a file required when the detection device performs the detection work, a task of loading the vessel from the preparation device to a vessel transfer module that transfers the vessel, and a task of transferring the loaded vessel through the vessel transfer module.

Wherein the plurality of task locations included in the device mark representing the detection device may include a task location indicating at least one task among a task of opening a cover of the detection device, a task of performing the detection work, a task of exhausting air in an internal space in which the detection device is placed, a task of loading the vessel from the detection device to a vessel transfer module that transfers the vessel, and a task of transferring the loaded vessel through the vessel transfer module.

Wherein the plurality of device marks may further include a device mark representing a sealing module that seals an upper surface of the vessel at which the preparation work has been completed.

Wherein the processor may execute the at least one instruction to cause the display unit to display the device mark representing the sealing module between the device mark representing the preparation device and the device mark representing the detection device.

Wherein a plurality of task locations included in the device mark representing the sealing module may include a task location indicating at least one task among a task of loading the vessel from the vessel transfer module that transfers the vessel to the sealing module, a task of sealing the loaded vessel, a task of loading the vessel from the sealing module to the vessel transfer module, and a task of transferring the loaded vessel through the vessel transfer module.

Wherein the processor may execute the at least one instruction to cause the display unit to display information obtained from a device represented by the device mark on the device mark.

Wherein the information obtained from the device represented by the device mark, which is displayed on the device mark, may include at least one of a remaining time of a task to be performed by the device represented by the device mark, an operating status of the device, an open/closed state of a door of the device, information on a loading status of consumables in the device, a temperature of the device, and a temperature of the vessel accommodated in the device.

Wherein the processor may execute the at least one instruction to cause the display unit to display an operating status of a fan module for exhausting air in an internal space where the detection device is placed.

Wherein the processor may execute the at least one instruction to cause the display unit to display an open/closed state of a door provided on a housing in which at least one of devices represented by the device marks is placed.

In accordance with another aspect of the present disclosure, there is provided a modular molecular diagnostic system comprising: a preparation device for performing a preparation work for detection of a target nucleic acid molecule; a detection device for performing a detection work for the target nucleic acid molecule; and a display unit that displays a device mark representing the preparation device and a device mark representing the detection device, wherein the display unit displays a position of a vessel used in the preparation device or the detection device by positioning a vessel symbol representing the vessel on one of the device marks.

The modular molecular diagnostic system may further comprise a sealing module that seals an upper surface of the vessel at which the preparation work has been completed, wherein the display unit may further display a device mark representing the sealing module.

In accordance with another aspect of the present disclosure, there is provided a modular molecular diagnostic system comprising: a preparation device for performing a preparation work for detection of a target nucleic acid molecule; a detection device for performing a detection work for the target nucleic acid molecule; and a display unit that displays a device mark representing the preparation device and a device mark representing the detection device, wherein the display unit displays information about the status of a device represented by each device mark or information about a task that each device performs in a predetermined sequence on device marks.

The modular molecular diagnostic system may further comprise a sealing module that seals an upper surface of the vessel at which the preparation work has been completed, wherein the display unit further displays a device mark representing the sealing module.

In accordance with another aspect of the present disclosure, there is provided a method for displaying a position of a vessel used in a detection work of a target nucleic acid molecule, which is performed by a vessel position display device, the method comprising: positioning a vessel symbol representing a vessel on one of a plurality of device marks, wherein the plurality of device marks include a device mark representing a preparation device that performs a preparation work for detecting a target nucleic acid molecule of an analysis sample and a device mark representing a detection device that performs a detection work for detecting the target nucleic acid molecule.

In accordance with another aspect of the present disclosure, there is provided a vessel position display method comprising: a preparation step of performing a preparation work for detecting a target nucleic acid molecule; a detection step of performing a detection work for the target nucleic acid molecule; and a display step of displaying a mark representing the preparation step and a mark representing the detection step, wherein the method further includes a step of positioning a vessel symbol representing a vessel used in the preparation step or the detection step on one of the marks.

In accordance with another aspect of the present disclosure, there is provided an information display method comprising: a preparation step of performing a preparation work for detecting a target nucleic acid molecule; a detection step of performing a detection work for the target nucleic acid molecule; and a display step of displaying a mark representing the preparation step and a mark representing the detection step, wherein the method further comprises a display step of displaying, on at least one of marks, information obtained from the step indicated by the marks.

### EFFECT OF INVENTION

According to one embodiment, a vessel symbol representing a specific vessel can be displayed on a display unit that displays device marks representing the preparation device and the detection device, respectively.

### BRIEF DESCRIPTION OF THE DRAWINGS

FIG. 1 is a front view showing a modular molecular diagnostic system according to one embodiment.
FIG. 2 is a right side view of the modular molecular diagnostic system according to one embodiment.
FIG. 3 is an exemplary diagram showing the operational connection of the modular molecular diagnostic system according to one embodiment of the present disclosure.
FIG. 4 is an internal front view showing an automated analysis system of the present disclosure.
FIG. 5 illustrates, as an example, a display unit 1600 of a modular molecular diagnostic system according to one embodiment, which shows a U/I 1610 for displaying the position of a vessel, a U/I 1620 for displaying the status of the modular molecular diagnostic system, and a drive software display U/I 1630 for displaying a driving software that drives the modular molecular diagnostic system.
FIG. 6 is an exemplary diagram illustrating a vessel position display U/I (User Interface) 2000 displayed on the display unit of the modular molecular diagnostic system according to one embodiment of the present disclosure, wherein a vessel symbol 2500 representing a vessel is positioned in one of a plurality of device marks 2100, 2200, 2400, 2700 representing a plurality of working devices.
FIG. 7 is an exemplary diagram illustrating a vessel position display U/I 3000 displayed on the display unit of the modular molecular diagnostic system according to one embodiment of the present disclosure, wherein a vessel symbol representing a vessel is indicated in a task location displayed as included in a device mark.
FIG. 8 is an exemplary diagram illustrating a vessel position display U/I 4000 displayed on the display unit of the modular molecular diagnostic system according to one embodiment of the present disclosure, wherein vessel symbols 4500-a, 4500-b corresponding to a plurality of vessels are displayed on a device mark.
FIG. 9 is an exemplary diagram illustrating a vessel position display U/I 5000, displayed on the display unit of the modular molecular diagnostic system according to one embodiment of the present disclosure, which displays a plurality of vessel positions in device marks and task locations.
FIG. 10 is an exemplary diagram illustrating an information display U/I 6000 that displays information obtained from the devices constituting the modular molecular diagnostic system, which is displayed on the display unit of the modular molecular diagnostic system according to one embodiment.
FIG. 11 is an exemplary diagram illustrating a vessel position display U/I 7000 that displays information obtained from the devices constituting the modular molecular diagnostic system and displays the position of a vessel, which is displayed on the display unit of the modular molecular diagnostic system according to one embodiment.
FIG. 12 is a flowchart of a vessel position display method performed by the vessel position display device according to one embodiment of the present disclosure.
FIG. 13 is a flowchart of an information display method performed by the information display device according to one embodiment.

### MODE FOR CARRYING OUT THE INVENTION

The advantages and features of the embodiments and the methods of accomplishing the embodiments will be clearly understood from the following description taken in conjunction with the accompanying drawings. However, embodiments are not limited to those embodiments described, as embodiments may be implemented in various forms. It should be noted that the present embodiments are provided to make a full disclosure and also to allow those skilled in the art to know the full range of the embodiments. Therefore, the embodiments are to be defined only by the scope of the appended claims.

In describing embodiments of the present invention, if it is considered that a detailed description of a known function or configuration may unnecessarily obscure the gist of the present invention, the detailed description will be omitted. In addition, the terms described below are terms defined in consideration of functions in the embodiments of the present invention, the terms may vary according to the intention or precedent of a technician working in the field, the emergence of new technologies, and the like. Therefore, the terms used in the present disclosure should be defined based on the meaning of the terms and the overall contents of the present disclosure, not just the name of the terms.

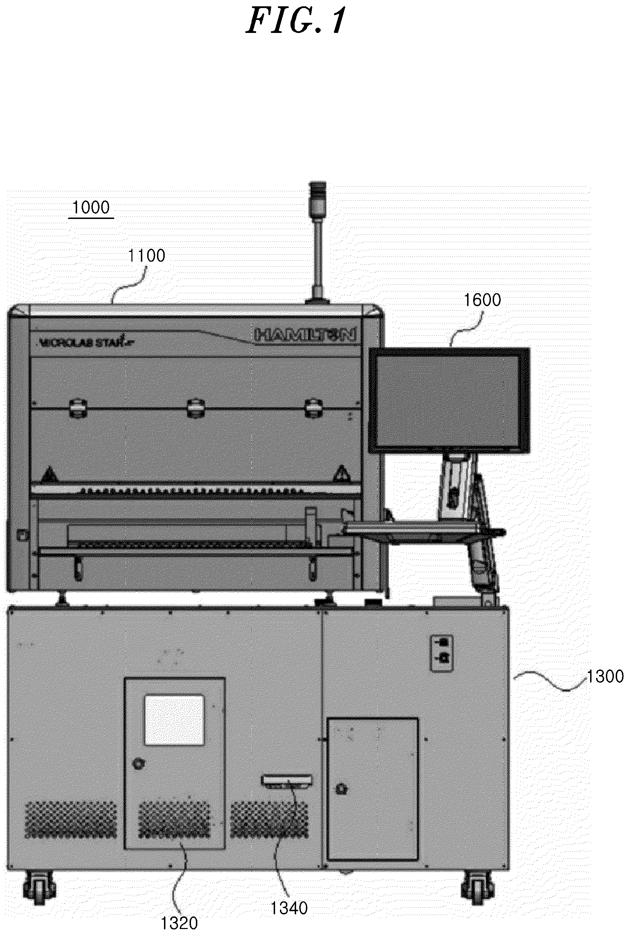

Before explaining FIG. 1, the terms used herein will be described.

The term "sample" refers to a substance that contains or is presumed to contain an analyte (which will be described later).

The sample may include biological samples (e.g., cells, tissues, and body fluids) and non-biological samples (e.g., food, water, and soil).

Examples of biological samples may include viruses, bacteria, tissues, cells, blood (including whole blood, plasma, and serum), lymph, bone marrow fluid, sputum, swabs, aspirations, bronchial lavage fluid, bronchoalveolar lavage fluid, nasal lavage fluid, milk, urine, feces, eye fluid, saliva, semen, brain extracts, spinal fluid (SCF), joint fluid, appendix, spleen and tonsil tissue extracts, amniotic fluid, or ascites. In addition, the examples of biological samples may also include naturally occurring nucleic acid molecules isolated from a biological source or synthetic nucleic acid molecules. However, the biological samples are not limited to the examples described above.

The aforementioned samples may have substances added to them for purpose such as preservation, processing, or detection. For example, the sample may have additional substances such as an amplification reagent, a detection reagent, a preservative, water, deionized water, saline solution, a pH buffer, an acidic solution, or a basic solution. The term "sample" used herein may refer to a sample to which the aforementioned additional substances have not been added, as well as a sample to which the aforementioned additional substances have been added.

Meanwhile, the aforementioned sample may be referred to variously depending on the stage of molecular diagnosis. For example, the term "raw sample" may refer to a sample before being processed in a first device to be described later. In addition, the term "analysis sample" may refer to intermediates including analytes produced during various steps of processing a sample for analysis.

Meanwhile, the term "sample" mentioned above may be used interchangeably with the term "specimen." In this case, the term "specimen" itself refers to a subject to be analyzed. More specifically, the term "specimen" may include sputum, blood, urine, stool, etc.

Meanwhile, the analyte includes an antigen, an antibody, an enzyme, or a nucleic acid. In a specific embodiment, the analyte includes a nucleic acid. In this case, the nucleic acid can be extracted through a known nucleic acid extraction process (reference: Sambrook, J. et al., Molecular Cloning. A Laboratory Manual, 3rd ed. Cold Spring Harbor Press (2001)). The nucleic acid extraction process may vary depending on the type of sample. In addition, when the extracted nucleic acid is RNA, a reverse transcription process for synthesizing cDNA may be additionally performed (see: Sambrook, J. et al., Molecular Cloning. A Laboratory Manual, 3rd ed. Cold Spring Harbor Press (2001)).

The term "vessel" used herein refers to a space that accommodates a substance used in a preparation device and an analysis device. The substance generally includes a solution. The vessel may be used as a "sample vessel" or a "reaction vessel" containing an analysis sample. In addition, in the present specification, the space that accommodates a substance used in the preparation device and the analysis device may be used as a "container" or a "carrier." "Vessel", "container", and "carrier" are not specifically distinguished, but may be selectively used depending on the device used, the shape, or the substance accommodated therein.

In addition, the vessel refers to a container used for nucleic acid extraction, composition of amplification reaction mixture, and amplification reaction setup (e.g., PCR setup) performed in the preparation device. In other words, a specimen, one or more extraction reagents, one or more compositions for reaction mixture, an analysis sample (master mix) that is a mixture of the extracted nucleic acid and the reaction mixture may be accommodated in the vessel, and an analysis sample intended for reaction, performed through the analysis device, may be dispensed and accommodated in a reaction vessel. In one embodiment of the present disclosure, the vessel includes a tube, a tube strip, and the like. In another embodiment of the present disclosure, the vessel may include a cartridge, a well plate, and the like.

The vessel may be of various sizes depending on the substance to be accommodated, and various means for storing or holding the vessel may be prepared according to the various sizes of the vessel. The means for holding the vessel may include a carrier, a rack, an adapter, etc., and one or more vessels may be inserted and stored in each means.

In one embodiment of the present disclosure, the vessel may include a cap. In another embodiment of the present disclosure, the vessel may be sealed using a film or the like.

The term "reaction vessel" as used herein refers to a sample vessel that can be accommodated in a sample holder of the analysis device. The reaction vessel accommodates a specified volume of an analysis sample containing a target nucleic acid or a specified volume of an analysis sample containing no target nucleic acid, and may be accommodated in the sample holder and used for a reaction (e.g., amplification) or detection (e.g., fluorescence signal).

The reaction vessel described in the present specification is exemplified as a tube capable of accommodating an analysis sample, but reaction vessels of various materials and shapes may be used depending on the shape of the reaction region. The reaction vessel is inserted into a well formed in the reaction region so that a reaction cycle of heating and cooling can be performed. In other words, the "reaction vessel" refers to a closed space where a reaction takes place.

The reaction vessel includes one or more reaction vessels. The reaction vessel refers to a unit that can accommodate an analysis sample (e.g., an analyte or a reaction mixture). A test tube, an amplification tube, a strip tube, a well plate, and a multi-well PCR plate are each one embodiment of the reaction vessel including one or more reaction vessels.

In one embodiment of the present disclosure, one or more reaction vessels may be mounted on the sample holder.

In another embodiment of the present disclosure, one or more reaction vessels are accommodated in a multi-well plate (hereinafter referred to as a "well plate"). The well plate that accommodates one or more reaction vessels may be mounted on the sample holder.

In another embodiment of the present disclosure, the reaction vessel is a well plate capable of accommodating an analysis sample in one or more wells. The well plate accommodating an analysis sample in one or more wells may be mounted on the sample holder.

The embodiments of the reaction vessel described above are some of the preferred embodiments of the present disclosure. Therefore, it is obvious that the reaction vessel can be implemented in various ways according to other embodiments. Hereinafter, with reference to the drawings, various embodiments of the present disclosure will be described.

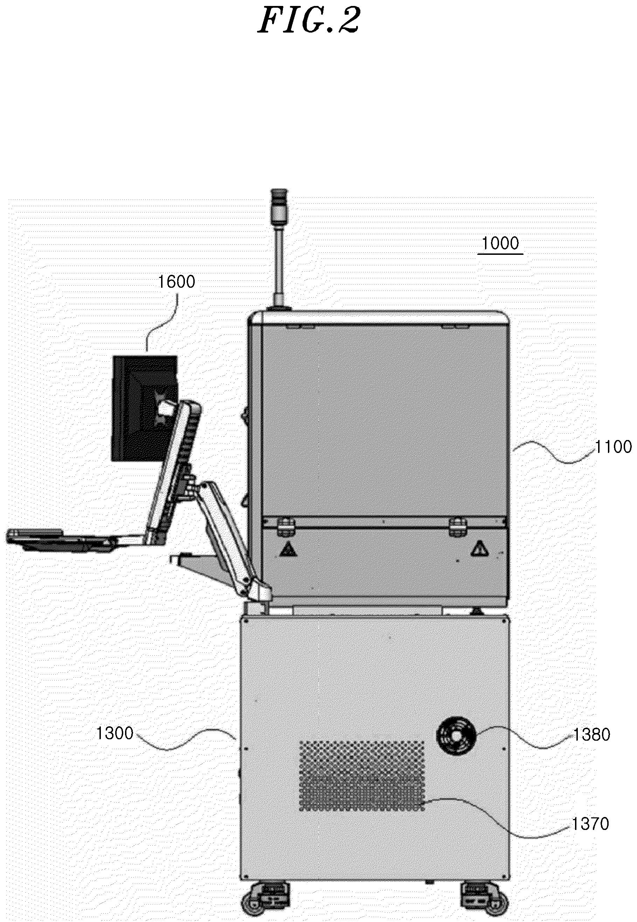

FIG. 1 is a front view showing a modular molecular diagnostic system according to one embodiment, and FIG. 2 is a right side view of the modular molecular diagnostic system according to one embodiment. As shown in FIGS. 1 and 2, in one embodiment, the modular molecular diagnostic system 1000 includes a preparation device 1100 and an enclosure 1300.

In one embodiment of the present disclosure, the preparation device 1100 may be configured to be positioned on top of the enclosure 1300.

In another embodiment of the present disclosure, the preparation device 1100 may be configured to be positioned on the side of the enclosure 1300.

In still another embodiment of the present disclosure, the preparation device 1100 may be configured to be located at the rear of the enclosure 1300.

In still another embodiment of the present disclosure, the preparation device 1100 may be configured to be located at the front of the enclosure 1300.

In still another embodiment of the present disclosure, the preparation device 1100 may be configured to be located inside the enclosure 1300.

In still another embodiment of the present disclosure, the preparation device 1100 may be configured to be positioned at the bottom of the enclosure 1300.

The enclosure 1300 may accommodate at least one of the preparation device 1100, an analysis device 1200, and a vessel transfer module 1400.

In one embodiment of the present disclosure, the enclosure 1300 may accommodate one or more analysis devices 1200 and the vessel transfer module 1400.

In another embodiment of the present disclosure, the enclosure 1300 may accommodate one or more analysis devices 1200.

In still another embodiment of the present disclosure, the enclosure 1300 may accommodate the vessel transfer module 1400.

In still another embodiment of the present disclosure, the enclosure 1300 may accommodate the preparation device 1100 and the vessel transfer module 1400.

In still another embodiment of the present disclosure, the enclosure 1300 may accommodate the preparation device 1100.

The enclosure 1300 can provide an operational connection between one or more devices accommodated therein and devices located outside.

In one embodiment of the present disclosure, the enclosure 1300 encloses a single space. When the enclosure 1300 encloses a single space, at least one of the preparation device 1100, the analysis device 1200, and the vessel transfer module 1400 can be located in the single space.

In another embodiment of the present disclosure, the enclosure 1300 encloses a plurality of spaces. When the enclosure 1300 encloses a plurality of spaces, at least one of the preparation device 1100, the analysis device 1200, and the vessel transfer module 1400 can be located in one of the plurality of spaces, or in two or more of the plurality of spaces.

For example, in the enclosure 1300 with a single space, the analysis device 1200 and the vessel transfer module 1400 can be located in the same space.

For example, in the enclosure 1300 with a plurality of spaces, the analysis device 1200 and the vessel transfer module 1400 can be located in different spaces.

In one embodiment of the present disclosure, the analysis device 1200 and the vessel transfer module 1400 may be positioned inside the enclosure 1300, while the preparation device 1100 is positioned outside the enclosure 1300.

The enclosure 1300 is formed with a defined passage through which the vessel transfer module 1400 moves to transfer a vessel 1500 to be provided from the preparation device 1100 to the analysis device 1200. The defined passage may be located on any one of the outer surfaces of the enclosure 1300.

The vessel transfer module 1400 can be programmed to transfer the vessel 1500 from the preparation device 1100 through the defined passage.

Accordingly, it is preferable that each defined passage is located at a distance within a range that allows the vessel transfer module 1400 to move the vessel 1500.

The enclosure 1300 is not sealed and may have an air vent 1370.

At least one selected from the group consisting of the preparation device 1100, the analysis device 1200, and the vessel transfer module 1400 may be positioned in the enclosure 1300.

Further, a sealing module 1700 for sealing an upper surface inlet of the vessel 1500 may be positioned in the enclosure 1300.

In addition, a reaction vessel recovery box (not shown) for collecting the vessel 1500 for which analysis has been completed in the analysis device 1200 may be provided to the enclosure 1300.

In one embodiment of the present disclosure, the reaction vessel recovery box may be located inside the enclosure 1300.

In another embodiment of the present disclosure, the reaction vessel recovery box may be located outside the enclosure 1300. When located outside, the reaction vessel recovery box can collect the vessel 1500 as the vessel 1500 is transferred from the interior to the exterior of the enclosure 1300 through a recovery opening 1340 connecting the interior and exterior of the enclosure 1300.

For the movement of the vessel 1500, a conveyor (not shown) may be installed in the recovery opening 1340 connecting the interior and exterior of the enclosure 1300. When the vessel transfer module 1400 puts the vessel 1500 down on the inner conveyor of the enclosure 1300, the vessel 1500 may be moved by the conveyor and received in the vessel recovery box located outside the enclosure 1300.

The vessel recovery box can be emptied of one or more vessels 1500 received therein by a user.

In one embodiment of the present disclosure, the enclosure 1300 is not completely sealed and is implemented to allow ventilation. To this end, the enclosure 1300 includes an environmental control means, and the environmental control means may include a ventilation hole, an exhaust hole, a fan, a temperature control means, a humidity control means, an air filter, and the like.

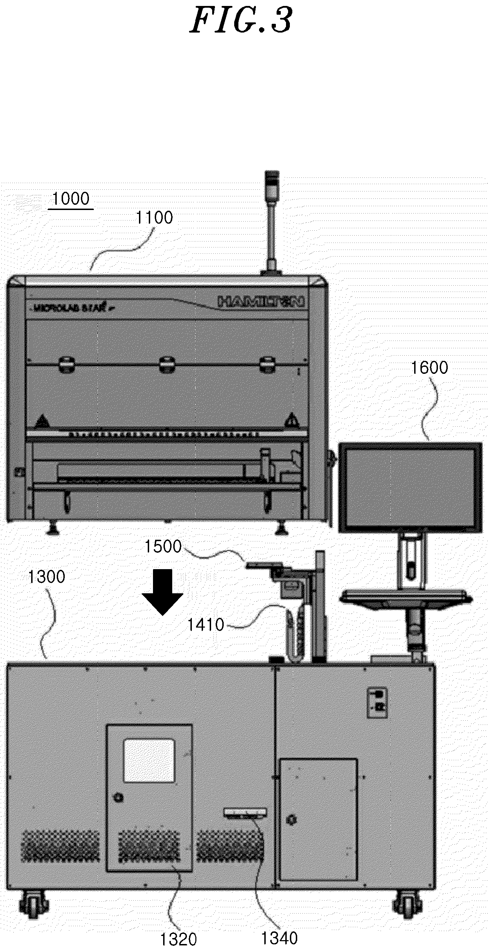

FIG. 3 is an exemplary diagram showing the operational connection of the modular molecular diagnostic system according to one embodiment of the present disclosure. As illustrated in FIG. 3, the preparation device 1100 is located on top of the enclosure 1300.

The preparation device 1100 may be independently installed and operated for preparing an analysis sample, and according to one embodiment of the present disclosure, it may be operatively connected to the enclosure 1300 to be used as the modular molecular diagnostic system 1000.

According to one embodiment of the present disclosure, when the preparation device 1100 is combined with the enclosure 1300 and used as the modular molecular diagnostic system 1000, the preparation device 1100 may have an opening so that a lift module 1410 provided in the enclosure 1300 can move into the interior of the preparation device 1100.

When the lift module 1410 is moved in the interior of the preparation device 1100, the preparation device 1100 mounts a plate (not shown) that holds a vessel 1500 accommodating an analysis sample onto the lift module 1410. The lift module 1410 moves the mounted vessel 1500 or plate into the interior of the enclosure 1300.

According to one embodiment of the present disclosure, when the preparation device 1100 is positioned on top of the enclosure 1300, the preparation device 1100 and the enclosure 1300 may be coupled through a coupling mechanism (not shown).

In one embodiment of the present disclosure, when at least one of the standalone preparation device 1100 and the standalone analysis device 1200 is operatively connected to the enclosure 1300 and used as the modular molecular diagnostic system 1000, the standalone preparation device 1100 and the standalone analysis device 1200 are commercially available devices and/or devices that have been approved as standalone devices.

Accordingly, even when the standalone preparation device 1100 or the standalone analysis device 1200 is operatively connected to the enclosure 1300 and used as the modular molecular diagnostic system 1000, no separate permit is required, or partial modifications may be made.

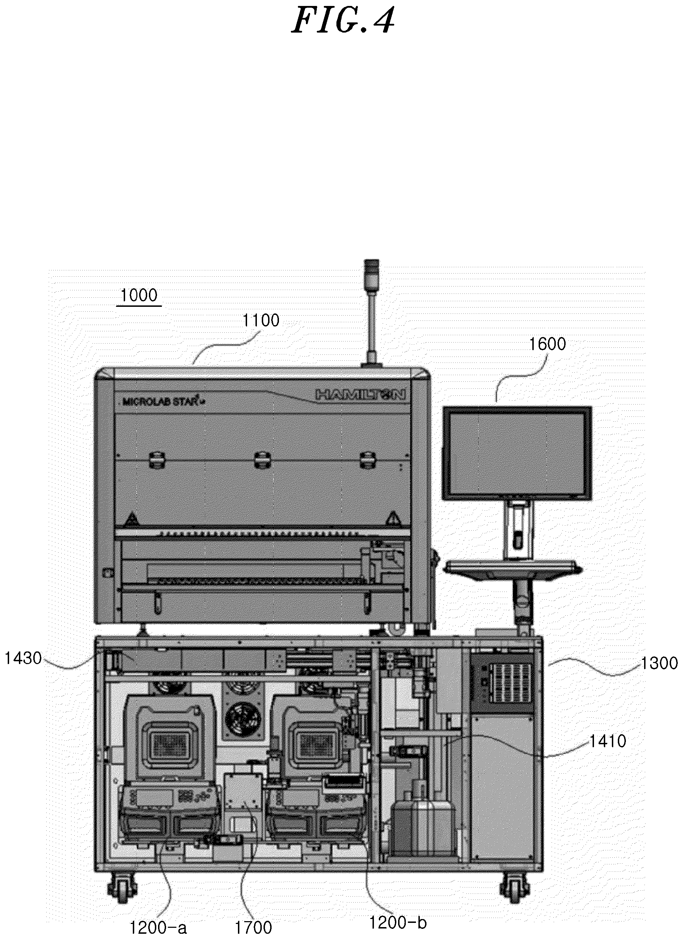

FIG. 4 is an internal front view showing an automated analysis system of the present disclosure. As shown in FIG. 4, analysis devices 1200-a, 1200-b are located in the lower portion of the enclosure 1300.

In one embodiment of the present disclosure, the enclosure 1300 includes the vessel transfer module 1400 for providing a reaction vessel 1500 prepared in the preparation device 1100 to the analysis devices 1200-a, 1200-b.

The vessel transfer module 1400 is a robot module, and includes, in particular, a lift module 1410 and a crane module 1430.

In one embodiment of the present disclosure, the vessel transfer module is configured to include the lift module 1410 and the crane module 1430.

In another embodiment of the present disclosure, the vessel transfer module is configured to include the lift module 1410.

In still another embodiment of the present disclosure, the vessel transfer module is configured to include the crane module 1430.

In still another embodiment of the present disclosure, the vessel transfer module is configured to include a robot arm (not shown).

In still another embodiment of the present disclosure, the vessel transfer module may include a mechanical device capable of transferring the reaction vessel 1500.

In one embodiment of the present disclosure, the lift module 1410 and the crane module 1430 may be located inside the enclosure 1300.

The lift module 1410 and the crane module 1430 included in the enclosure 1300 are robotic modules. The robotic modules move the reaction vessel 1500 under the control of a control module (not shown) included in the modular molecular diagnostic system.

A sealing module 1700 for sealing the inlet of the reaction vessel 1500 may be positioned in the enclosure 1300.

At least one analysis device 1200-a, 1200-b for analyzing an analysis sample received in the reaction vessel 1500 may be positioned in the enclosure 1300.

A solution recovery box (not shown) for recovering various solutions used for the preparation of an analysis sample in the preparation device 1100 may be positioned in the enclosure 1300.

The enclosure 1300 may have a reaction vessel recovery box (not shown) for recovering a reaction vessel 1500 for which analysis has been completed in the analysis device 1200-a, 1200-b.

The enclosure 1300 includes a control module (not shown) for transmitting and/or receiving data from a plurality of devices operatively connected to the modular molecular diagnostic system 1000.

In one embodiment of the present disclosure, the control module may be connected to a communication channel to be operatively connected to at least one of the preparation device 1100, the analysis device 1200, the vessel transfer module 1400, and/or the sealing module 1700. For example, the communication channel may be connected in wireless and/or wired manner.

In one embodiment of the present disclosure, the control module can control the preparation device, the analysis device, and the vessel transfer module to operate in a timely manner. The control module controls the preparation device, the analysis device, and the vessel transfer module so that the preparation of an analysis sample and the analysis thereof using the preparation device and the analysis device of the present disclosure, which are standalone devices, can be automatically performed. Specifically, once the preparation of the analysis sample in the preparation device is completed, the control module controls the vessel transfer module to transfer the analysis sample to the analysis device. In addition, the control module provides a necessary signal to the analysis device to load the analysis sample into the analysis device, thereby loading the analysis sample and causing the analysis device to start the analysis.

In addition, in one embodiment of the present disclosure, the control module may provide an external signal required for the operation of the standalone devices. The external signal required for the operation may be, for example, a signal that causes the analysis device to be in a state capable of receiving an analysis sample, a signal that causes the analysis device to start analysis, or a signal that causes the sealing module to start a sealing operation.

In this way, the control module not only transmits signals between the standalone devices to enable the analysis sample to move from the preparation device to the analysis device, but also provides signals that previously required external manual input by a user or the like in a timely manner so that the standalone devices can independently perform unit operations, thereby enabling the implementation of a full automation system.

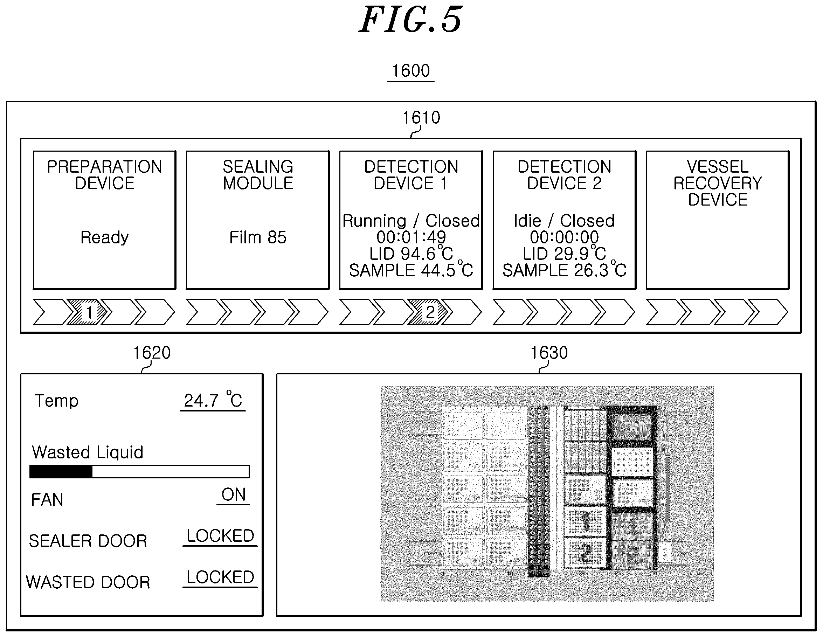

FIG. 5 illustrates, as an example, a display unit 1600 of a modular molecular diagnostic system according to one embodiment, which shows a U/I 1610 for displaying the position of a vessel, a U/I 1620 for displaying the status of the modular molecular diagnostic system, and a drive software display U/I 1630 for displaying a driving software that drives the modular molecular diagnostic system. Such a configuration may be displayed on a part of the display screen of the modular molecular diagnostic system 1000. However, the configuration illustrated in FIG. 5 is merely exemplary.

Referring to FIG. 5, the display unit 1600 may provide a user interface (U/I) for the modular molecular diagnostic system 1000 or devices included therein. The user interface may be provided as a combination of a vessel position display U/I 1610 for displaying the position of the vessel described above, a status display U/I 1620 for displaying the status of the modular molecular diagnostic system, and a driving software display U/I 1630 for displaying a driving software that drives the modular molecular diagnostic system. Through these user interfaces, a user can monitor or control the modular molecular diagnostic system 1000 or devices included therein. Hereinafter, the interfaces 1610, 1620, 1630 that can be used for the user interface will be described.

The display unit 1600 according to one embodiment may provide the vessel position display U/I 1610 and the status display U/I 1620 for displaying the status of the modular molecular diagnostic system, for monitoring the modular molecular diagnostic system.

According to one embodiment, the vessel position display U/I 1610 is a U/I that indicates which component device in the modular molecular diagnostic system 1000 a vessel is located in. The vessel position display U/I 1610 may be displayed using at least a portion of the display unit 1600. Referring to FIG. 5, the vessel position display U/I 1610 may display regions corresponding to component devices that may be used in the modular molecular diagnostic system 100, such as the preparation device, the sealing module, the detection device, and the vessel recovery device. In the following description, a region or section displayed in the U/I on the display to correspond to a component device is defined as a "device mark" representing the component device. The device mark may be represented on the display in any shape or form, provided it allows independent identification of a component device of the modular molecular diagnostic system 100. In addition, the sequence or layout of the device marks on the display may be set regardless of the arrangement of actual devices inside the modular molecular diagnostic system.

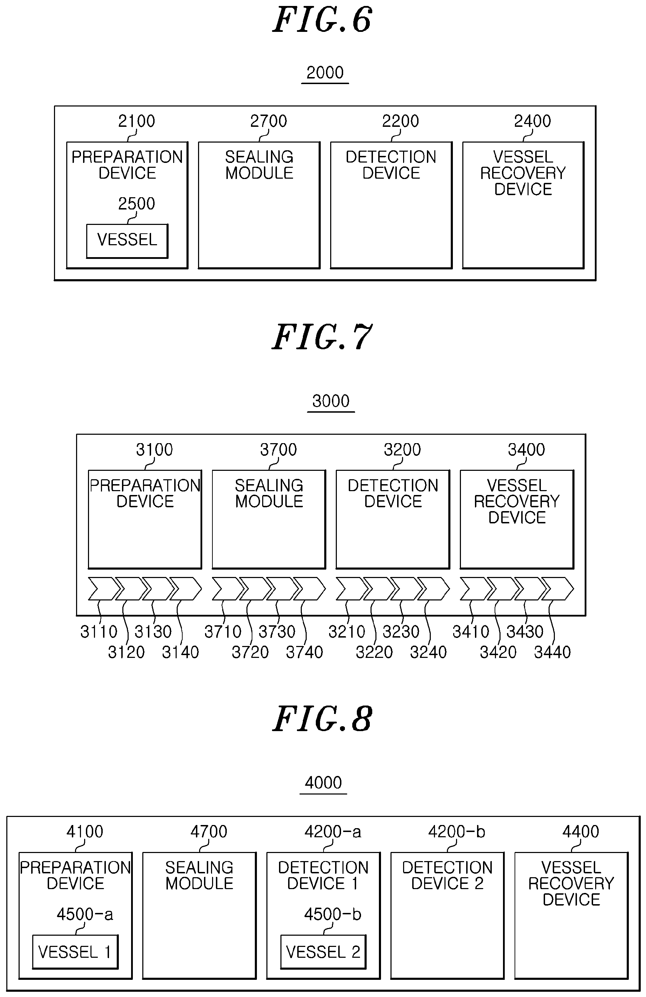

Further, referring to FIG. 5, the vessel position display U/I 1610 according to one implementation example may display information obtained from the devices represented by the device marks on the respective device marks. In addition, the vessel position display U/I 1610 may indicate not only the position of the vessel, but also the task status using the task location. The obtained information, the task location, and the task will be described later with reference to FIGS. 6 to 11, which are drawings regarding the vessel position display U/I.

Meanwhile, the display unit 1600 according to one embodiment may provide the status display U/I 1620 for monitoring the modular molecular diagnostic system in addition to the vessel position display U/I 1610 described above.

According to one embodiment, the status display U/I 1620 may display the operating status of the modular molecular diagnostic system 1000 or devices included therein. Referring to FIG. 5, the status display U/I 1620 may display the temperature measured in a specific part of the modular molecular diagnostic system 100, the loading status of a vessel recovery box for collecting used vessels, the operating status of a fan, the open/close state of a door installed in the aforementioned enclosure, and the like. When the vessel position display U/I 1610 according to one embodiment displays information obtained from the preparation device, the sealing module, the detection device, and the vessel recovery device, the status display U/I 1620 may display information acquired from devices or structures other than these.

Meanwhile, the display unit 1600 according to one embodiment may provide the driving software display U/I 1630 for displaying a driving software that drives the modular molecular diagnostic system to monitor or control the modular molecular diagnostic system. As described above, in the modular molecular diagnostic system 1000 according to one embodiment, the standalone preparation device 1100 and the standalone analysis device 1200 can be operatively connected. In this case, when the standalone preparation device 1100 and the standalone analysis device 1200 have software for driving individual devices, the display unit 1600 according to one embodiment can display a screen for the used software by using the driving software display U/I 1630 provided by the display unit 1600.

Referring to FIGS. 6 to 11, additional embodiments of the aforementioned vessel position display U/I 1610 will be described. The vessel position display U/I described below may be displayed on at least a portion of the display unit of the modular molecular diagnostic system to indicate the position of a vessel, similar to the embodiment of FIG. 5.

FIG. 6 is an exemplary diagram illustrating a vessel position display U/I (User Interface) 2000 displayed on the display unit of the modular molecular diagnostic system according to one embodiment of the present disclosure, wherein a vessel symbol 2500 representing a vessel is positioned in one of a plurality of device marks 2100, 2200, 2400, 2700 representing a plurality of working devices. This configuration may be displayed on a part of a screen displayed on the display unit of the modular molecular diagnostic system 1000. However, the configuration illustrated in FIG. 6 is merely exemplary.

A vessel position display device according to one embodiment includes a display unit, a memory storing at least one instruction, and a processor. In this case, the vessel position display U/I may be output through the display unit, and the processor may execute the at least one instruction to display on the display unit a vessel symbol corresponding to a specific vessel and representing the specific vessel in a device mark representing a device where the vessel is located, wherein the device mark includes a device mark 2100 representing a preparation device performing a preparation work for detecting a target nucleic acid molecule and a device mark 2200 representing a detection device performing a detection work for the target nucleic acid molecule. The vessel position display U/I 2000 according to one embodiment may indicate a device where a vessel is located on the display unit, and in this case, a mark corresponding to the vessel, that is, a vessel symbol, may be displayed inside or near the device mark representing the device where the vessel is located. In this case, the vessel symbol may be represented in any manner that indicates the vessel, without restrictions on the format and shape, such as letters, graphics, shapes, colors, and check marks representing the vessel.

In addition, the device mark may further include a device mark 2700 representing a sealing module for sealing the upper surface of a vessel for which the preparation work has been completed. In this case, the device mark 2700 representing the sealing module may be displayed between the device mark 2100 representing the preparation device and the device mark 2200 representing the detection device.

In addition, the device mark may further include a device mark (not shown) representing a specific discharge port where the vessel transfer module transferring the vessel 2500 is positioned to discharge the vessel, for which the detection work has been completed, out of a specific housing in which the detection device and the sealing module are arranged. The device mark may be used to indicate a certain location on the path along which the vessel is moved through the vessel transfer module transferring the vessel. In this case, the arrangement order of the device marks displayed on the display unit may be displayed to indicate the order in which the vessel is moved. However, since the modular molecular diagnostic system 1000 according to one embodiment may be configured to include standalone devices capable of independent operation as described above, the vessel may be loaded into the component device of the modular molecular diagnostic system 1000 for use regardless of the order or arrangement of the device marks displayed on the display unit. In this case, the arrangement of the device marks displayed on the display unit may be unrelated to the order in which the vessel is moved.

FIG. 7 is an exemplary diagram illustrating a vessel position display U/I 3000 displayed on the display unit of the modular molecular diagnostic system according to one embodiment of the present disclosure, wherein a vessel symbol representing a vessel is indicated in a task location displayed as included in a device mark. This configuration may be displayed on a portion of a screen displayed on the display unit of then modular molecular diagnostic system 1000. However, the configuration illustrated in FIG. 7 is merely exemplary.

In the preparation device of the modular molecular diagnostic system connected to the vessel position display device according to one embodiment, a plurality of tasks are performed in a specific sequence, and a device mark 3100 representing the preparation device may display task locations 3110, 3120, 3130, 3140 indicating the respective tasks. In this case, in the display unit of the modular molecular diagnostic system, a vessel symbol may be displayed at any one of a plurality of task locations indicating the respective tasks.

In this case, the plurality of tasks may include at least one of a task of generating a file required when the detection device performs the detection work, a task of loading the vessel from the preparation device to the vessel transfer module that transfers the vessel, and a task of transferring the loaded vessel through the vessel transfer module. For example, the preparation device may accommodate a plurality of vessels (a first vessel and a second vessel) to perform a preparation work, and in this case, the task locations 3110, 3120, 3130, 3140 may respectively be a task location 3110 corresponding to a task of generating a file required when the detection device performs a detection work for the first vessel, a task location 3120 corresponding to a task of generating a file required when the detection device performs a detection work for the second vessel, a task location 3130 corresponding to a task of loading the vessel from the preparation device to the vessel transfer module that transfers the vessel, and a task location 3140 corresponding to a task of transferring the loaded vessel through the vessel transfer module. Referring to FIG. 7, it can be seen that a vessel symbol representing a vessel is displayed at the task location 3120 with color highlighting. This indicates that the second vessel is located in the preparation device, and that the preparation device is performing a specific task, wherein the specific task is, for example, a "task of generating a file required when the detection device performs a detection work for the second vessel." With respect to the above tasks, the tasks matching the respective task locations may be notified in advance through instructions that can be distributed to users in advance, etc.

In this case, the plurality of task locations included in the device mark representing the preparation device may include a task location indicating at least one task among the task of generating a file required when the detection device performs the detection work, the task of loading the vessel from the preparation device to the vessel transfer module that transfers the vessel, and the task of transferring the loaded vessel through the vessel transfer module.

In addition, in the detection device of the modular molecular diagnostic system connected to the vessel position display device according to one embodiment, a plurality of tasks are performed in a specific sequence, and a device mark 3200 representing the detection device may display task locations 3210, 3220, 3230, 3240 indicating the respective tasks. In this case, on the display unit of the modular molecular diagnostic system, a vessel symbol may be displayed at the task location indicating the task being performed among the task locations indicating the respective tasks.

In this case, the plurality of tasks may include at least one of a task of opening a cover of the detection device, a task of performing the detection work, a task of exhausting air in an internal space where the detection device is placed, a task of loading the vessel from the detection device to the vessel transfer module that transfers the vessel, and a task of transferring the loaded vessel through the vessel transfer module.

In other words, the plurality of task locations included in the device mark representing the detection device may include a task location indicating at least one task among the task of opening the cover of the detection device, the task of performing the detection work, the task of exhausting air in the internal space in which the detection device is placed, the task of loading the vessel from the detection device to the vessel transfer module that transfers the vessel, and the task of transferring the loaded vessel through the vessel transfer module.

In addition, in the sealing module of the modular molecular diagnostic system connected to the vessel position display device according to one embodiment, a plurality of tasks are performed in a specific sequence, and a device mark 3700 representing the sealing module may display task locations 3710, 3720, 3730, 3740 indicating the respective tasks. In this case, on the display unit of the modular molecular diagnostic system, a vessel symbol may be displayed at the task location indicating the task being performed among the task locations indicating the respective tasks.

In this case, the plurality of tasks may include at least one of a task of loading the vessel from the vessel transfer module that transfers the vessel to the sealing module, a task of sealing the loaded vessel, a task of loading the vessel from the sealing module to the vessel transfer module, and a task of transferring the loaded vessel through the vessel transfer module.

In other words, the plurality of task locations included in the device mark representing the sealing module may include a task location indicating at least one task among the task of loading the vessel from the vessel transfer module that transfers the vessel to the sealing module, the task of sealing the loaded vessel, the task of loading the vessel from the sealing module to the vessel transfer module, and the task of transferring the loaded vessel through the vessel transfer module.

FIG. 8 is an exemplary diagram illustrating a vessel position display U/I 4000 displayed on the display unit of the modular molecular diagnostic system according to one embodiment of the present disclosure, wherein vessel symbols 4500-a, 4500-b corresponding to a plurality of vessels are displayed on a device mark. This configuration may be displayed on a portion of a screen displayed on the display unit of the modular molecular diagnostic system 1000. However, the configuration illustrated in FIG. 8 is merely exemplary.

Referring to FIG. 8, as described above, the modular molecular diagnostic system according to one embodiment may include a plurality of detection devices, and accordingly, the vessel position display U/I 4000 may display device marks 4200-a, 4200-b representing the plurality of detection devices.

Meanwhile, referring to FIG. 8, a first vessel symbol 4500-a representing a first vessel is displayed on a device mark 4100 representing the preparation device, and a second vessel symbol 4500-b representing a second vessel is displayed on a device mark 4200-a representing a first detection device. Through this, it can be seen that the first vessel is located in the preparation device, and the second vessel is located in the first detection device.

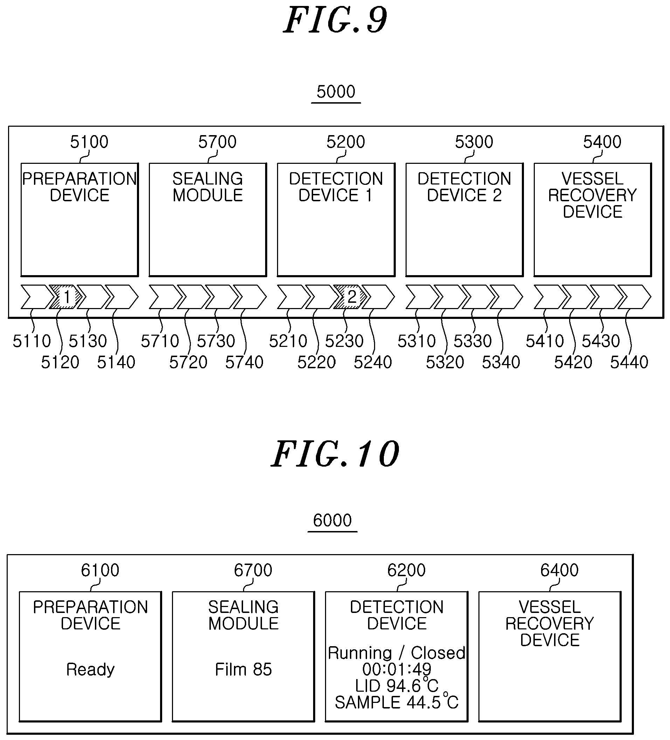

FIG. 9 is an exemplary diagram illustrating a vessel position display U/I 5000, displayed on the display unit of the modular molecular diagnostic system according to one embodiment of the present disclosure, which displays a plurality of vessel positions in device marks and task locations. This configuration may be displayed on a portion of a screen displayed on the display unit of the modular molecular diagnostic system 1000. However, the configuration illustrated in FIG. 9 is merely exemplary.

Referring to FIG. 9, it can be seen that the first vessel is positioned in the preparation device and the second vessel is positioned in the first detection device.

Further, referring to FIG. 9, it can be seen that each of device marks 5100, 5700, 5200, 5300, 5400 displayed on the vessel position display U/I 5000 includes matching task locations. The task locations may be preset to match the plurality of tasks described above, and the number and position of the task locations may be changed by user settings.

FIG. 10 is an exemplary diagram illustrating an information display U/I 6000 that displays information obtained from the devices constituting the modular molecular diagnostic system, which is displayed on the display unit of the modular molecular diagnostic system according to one embodiment. This configuration may be displayed on a portion of a screen displayed on the display unit of the modular molecular diagnostic system 1000. However, the configuration illustrated in FIG. 10 is merely exemplary.

The information display U/I 6000 according to one embodiment may be displayed on the display unit of the modular molecular diagnostic system 1000 as a substitute for the vessel position display U/I described with reference to FIGS. 5 to 9. The information display U/I and the vessel position display U/I according to one embodiment may be interchanged with each other according to user settings. In addition, the user can indirectly determine the position of the vessel by using information such as temperature and operating status displayed on the information display U/I, and can also obtain information by checking the contents displayed on the vessel position display U/I. Therefore, the two U/Is can be used without functional distinction.

An information display device according to one embodiment includes a display unit, a memory storing at least one instruction, and a processor. In this case, the information display U/I 6000 is displayed through the display unit, and the at least one instruction is executed by the processor so that a device mark 6100 representing a preparation device performing a preparation work for detection of a target nucleic acid molecule and a device mark 6200 representing a detection work for the target nucleic acid molecule are displayed on the display unit, and each of the device marks displays information obtained from the device represented by each of the device marks.

In this case, the information obtained from each of the above devices may be associated with the status of each of the above devices or with the tasks being performed by the corresponding device in a specific sequence.

In this case, the status of the device corresponding to each of the above device marks may include at least one of the remaining time of the task being performed by the corresponding device, the operating status of the corresponding device, the open/closed state of the door mounted on the corresponding device, information on the loading status of consumables accommodated in the corresponding device, the internal temperature of the corresponding device, and the temperature inside the vessel accommodated in the corresponding device.

In this case, the information display device according to one embodiment can display information obtained from the device on the device mark of the display unit by executing at least one instruction by the processor.

Referring to FIG. 10, the device mark 6100 representing the preparation device currently displays "Ready", which indicates a standby state without performing preparation work. In addition, the device mark 6700 representing the sealing module displays "85", which indicates the loading status of the film accommodated in the sealing module. Further, the device mark 6200 representing the detection device displays the operating status (Running), the open/closed state of the cover (Closed), and the temperatures of the detection device and the vessel.

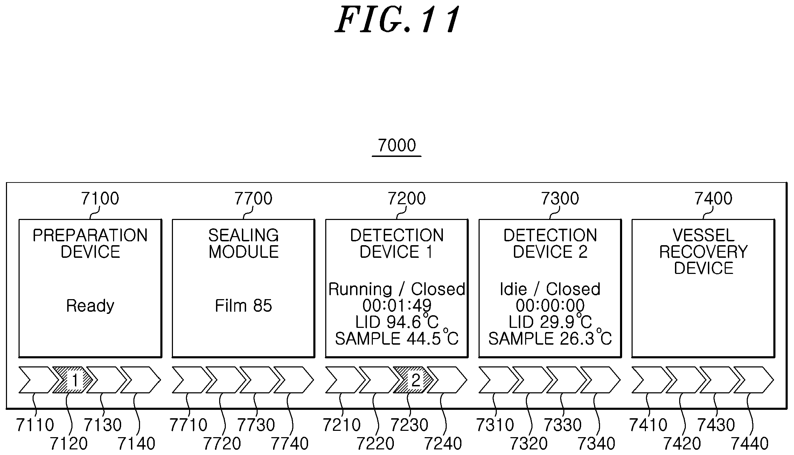

FIG. 11 is an exemplary diagram illustrating a vessel position display U/I 7000 that displays information obtained from the devices constituting the modular molecular diagnostic system and displays the position of a vessel, which is displayed on the display unit of the modular molecular diagnostic system according to one embodiment. This configuration may be displayed on a portion of a screen displayed on the display unit of the modular molecular diagnostic system 1000. However, the configuration illustrated in FIG. 11 is merely exemplary.

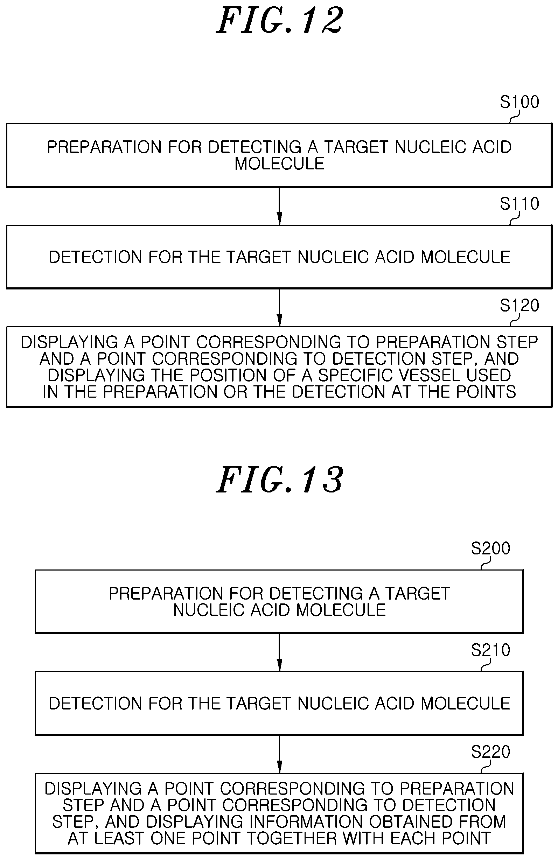

FIG. 12 is a flowchart of a vessel position display method performed by the vessel position display device according to one embodiment of the present disclosure.

The vessel position display method according to one embodiment includes a preparation step S100 of performing a preparation work for detecting a target nucleic acid molecule; a detection step S110 of performing a detection work for the target nucleic acid molecule; and a display step S120 of displaying a mark representing the preparation step and a mark representing the detection step, and displaying a vessel symbol representing a specific vessel used in the preparation work or the detection work on the marks.

FIG. 13 is a flowchart of an information display method performed by the information display device according to one embodiment.

The information display method according to one embodiment includes a preparation step S200 of performing a preparation work for detection of a target nucleic acid molecule; a detection step S210 of performing a detection work for the target nucleic acid molecule; and a display step S220 of displaying a mark representing the preparation step and a mark representing the detection step, and displaying information obtained from the step represented by at least one of the marks on the corresponding mark.

As described above, according to one embodiment, a vessel symbol representing a specific vessel can be displayed on the display unit that displays device marks corresponding to the preparation device and the detection device, respectively.

The above description is merely exemplary description of the technical scope of the present disclosure, and it will be understood by those skilled in the art that various changes and modifications can be made without departing from original characteristics of the present disclosure. Therefore, the embodiments disclosed in the present disclosure are intended to explain, not to limit, the technical scope of the present disclosure, and the technical scope of the present disclosure is not limited by the embodiments. The protection scope of the present disclosure should be interpreted based on the following claims and it should be appreciated that all technical scopes included within a range equivalent thereto are included in the protection scope of the present disclosure.

## Claims

1. A device for displaying a position of a vessel that accommodates an analysis sample, the device comprising:
a display unit;
a processor; and
a memory,
wherein the memory stores at least one instruction, executed by the processor to cause the display unit to display the position of the vessel by positioning a vessel symbol representing the vessel on one of a plurality of device marks, and
wherein the plurality of device marks include a device mark representing a preparation device that performs a preparation work for detecting a target nucleic acid molecule of the analysis sample and a device mark representing a detection device that performs a detection work for detecting the target nucleic acid molecule.

2. The device of claim 1, wherein each of the plurality of device marks includes a plurality of task locations indicating a plurality of tasks that each of devices represented by the plurality of device marks performs in accordance with a predetermined sequence, and
wherein the processor executes the at least one instruction to cause the display unit to display the position of the vessel by positioning the vessel symbol at one of the plurality of task locations.

3. The device of claim 2, wherein the plurality of task locations in the device mark representing the preparation device include a task location indicating at least one task among a task of generating a file required when the detection device performs the detection work, a task of loading the vessel from the preparation device to a vessel transfer module that transfers the vessel, and a task of transferring the loaded vessel through the vessel transfer module.

4. The device of claim 2, wherein the plurality of task locations in the device mark representing the detection device include a task location indicating at least one task among a task of opening a cover of the detection device, a task of performing the detection work, a task of exhausting air in an internal space in which the detection device is placed, a task of loading the vessel from the detection device to a vessel transfer module that transfers the vessel, and a task of transferring the loaded vessel by the vessel transfer module.

5. The device of claim 2, wherein the plurality of device marks further include a device mark representing a sealing module that seals an upper surface of the vessel at which the preparation work has been completed.

6. The device of claim 5, wherein the processor executes the at least one instruction to cause the display unit to display the device mark representing the sealing module between the device mark representing the preparation device and the device mark representing the detection device.

7. The device of claim 6, wherein the plurality of task locations in the device mark representing the sealing module include a task location indicating at least one task among a task of loading the vessel from the vessel transfer module that transfers the vessel to the sealing module, a task of sealing the loaded vessel, a task of loading the vessel from the sealing module to the vessel transfer module, and a task of transferring the loaded vessel by the vessel transfer module.

8. The device of claim 1, wherein the processor executes the at least one instruction to cause the display unit to display information obtained from a device represented by the device mark on the device mark.

9. The device of claim 8, wherein the information obtained from the device represented by the device mark, which is displayed on the device mark, includes at least one of a remaining time of a task to be performed by the device, an operating status of the device, an open/closed state of a door of the device, information on a loading status of consumables in the device, a temperature of the device, and a temperature of the vessel accommodated in the device.

10. The device of claim 1, wherein the processor executes the at least one instruction to cause the display unit to display an operating status of a fan module for exhausting air in an internal space where the detection device is placed.

11. The device of claim 5, wherein the processor executes the at least one instruction to cause the display unit to display an open/closed state of a door provided on a housing in which at least one of devices represented by the device marks is placed.

12. A modular molecular diagnostic system comprising:
a preparation device for performing a preparation work for detection of a target nucleic acid molecule;
a detection device for performing a detection work for the target nucleic acid molecule; and
a display unit that displays a device mark representing the preparation device and a device mark representing the detection device,
wherein the display unit displays a position of a vessel used in the preparation device or the detection device by positioning a vessel symbol representing the vessel on one of the device marks.

13. The modular molecular diagnostic system of claim 12, further comprising a sealing module that seals an upper surface of the vessel at which the preparation work has been completed, wherein the display unit further displays a device mark representing the sealing module.

14. A modular molecular diagnostic system comprising:
a preparation device for performing a preparation work for detection of a target nucleic acid molecule;
a detection device for performing a detection work for the target nucleic acid molecule; and
a display unit that displays a device mark representing the preparation device and a device mark representing the detection device,
wherein the display unit displays information about the status of a device represented by each device mark or information about a task that each device performs in a predetermined sequence on device marks.

15. The modular molecular diagnostic system of claim 14, further comprising a sealing module that seals an upper surface of the vessel at which the preparation work has been completed, wherein the display unit further displays a device mark representing the sealing module.

16. A method for displaying a position of a vessel used in a detection work of a target nucleic acid molecule, which is performed by a vessel position display device, the method comprising:
positioning a vessel symbol representing a vessel on one of a plurality of device marks,
wherein the plurality of device marks include a device mark representing a preparation device that performs a preparation work for detecting a target nucleic acid molecule of an analysis sample and a device mark representing a detection device that performs a detection work for detecting the target nucleic acid molecule.

17. A vessel position display method comprising:
a preparation step of performing a preparation work for detecting a target nucleic acid molecule;
a detection step of performing a detection work for the target nucleic acid molecule; and
a display step of displaying a mark representing the preparation step and a mark representing the detection step,
wherein the method further includes a step of positioning a vessel symbol representing a vessel used in the preparation step or the detection step on one of the marks.

18. An information display method comprising:
a preparation step of performing a preparation work for detecting a target nucleic acid molecule;
a detection step of performing a detection work for the target nucleic acid molecule; and
a display step of displaying a mark representing the preparation step and a mark representing the detection step,
wherein the method further comprises a display step of displaying, on at least one of marks, information obtained from the step indicated by the marks.

19. A computer program stored in a non-transitory computer-readable storage medium, which is programmed to perform each step included in the method according to any one of claims 16 to 18.

20. A computer-readable recording medium storing a computer program programmed to perform each step included in the method according to any one of claims 16 to 18.
